Europäisches Patentamt

European Patent Office

Office européen des brevets

(11) Numéro de publication: **0 227 513
B1**

(12) **FASCICULE DE BREVET EUROPEEN**

(19)

(45) Date de publication du fascicule du brevet:
04.07.90

(21) Numéro de dépôt: 86402496.3

(22) Date de dépôt: 07.11.86

(51) Int. Cl.⁵: **A61F 13/15**

(54) **Change complet avec matelas absorbant ramifié et son procédé de fabrication.**

(30) Priorité: **19.11.85 FR 8517100**

(43) Date de publication de la demande:
**01.07.87 Bulletin 87/27**

(45) Mention de la délivrance du brevet:
**04.07.90 Bulletin 90/27**

(84) Etats contractants désignés:
**BE DE ES FR GB GR IT LU NL**

(56) Documents cités:
**DE-A- 2 550 438**

(73) Titulaire: **KAYSERSBERG SA, Route de Lapoutroie,
F-68240 Kaysersberg(FR)**

(72) Inventeur: **Mazars, Paul, Amfreville sur Iton,
F-27400 Louviers(FR)**

(74) Mandataire: **David, Daniel, KAYSERSBERG 54, avenue
Hoche, F-75008 Paris(FR)**

## Description

L'invention porte sur un change complet à usage unique, du type destiné à absorber et retenir les liquides corporels, de bébés ou adultes incontinents. Elle se rapporte en particulier à la forme du matelas absorbant incorporé dans le change.

Les changes complets comprennent de façon connue, un matelas absorbant constitué essentiellement de fibres cellulosiques obtenues par défibrage à sec de feuilles de pâte à papier, recouvert sur une première face d'une feuille imperméable aux liquides en matériau thermoplastique souple et résistant tel que le polyéthylène. Cette feuille forme la face externe du change ; elle assure à la fois une fonction de support et empêche les fuites de liquides susceptibles de traverser la matelas absorbant.

Le face opposée du matelas absorbant est recouverte d'une feuille perméable aux liquides, généralement un non-tissé. Cette feuille, définissant la face interne du change, assure le maintien des fibres du matelas en place et vient au contact de la peau de l'utilisateur. Elle est de nature à laisser passer aisément les liquides corporels afin de permettre leur rapide absorption par le matelas sous-jacent.

Les deux feuilles, perméable et imperméable, sont liées entre elles, bord à bord, de manière à enfermer complètement le matelas absorbant. Le change est de forme générale rectangulaire, il peut être replié sur lui-même longitudinalement dans la zone d'entrejambe de manière que sa dimension transversale soit réduite en cet endroit. Cet objectif est également atteint par des découpes arrondies représentant le contour du haut des cuisses.

Le largeur du change, dans le zone de tronc avant et la zone de tronc arrière, correspond sensiblement à la largeur du bassin de l'utilisateur auquel est destiné le change, afin que ce dernier, après mise en place, enveloppe le bassin aussi hermétiquement que possible pour éviter les fuites latérales.

Dans le but d'éviter les fuites, on doit faire en sorte que le matelas absorbant couvre la plus grande surface possible, et cela pour deux raisons au moins : la première tient à la capacité d'absorption du matelas qui est fonction du volume occupé par les fibres. La deuxième raison vient de ce que la matelas doit pouvoir capter, sur une surface étendue, les liquides migrant le long du change. Cela est vrai notamment pour les utilisateurs masculins.

Par ailleurs ce type de matelas de fibres présente l'inconvénient, outre celui d'être volumineux, qui est de posséder un faible pouvoir de diffusion des liquides. L'absorption reste localisée à la zone de pénétration si bien que la capacité totale d'absorption n'est pas utilisée de façon optimale.

On a déjà proposé d'y remédier en ménageant dans le matelas des lignes densifiées par compactage, orientées suivant des directions déterminées. Le brevet US 2788003 en décrit un exemple de réalisation. En effet, en comprimant le matelas localement, on réduit l'espacement entre les fibres et facilite la conduction par capillarité. Il est possible d'améliorer ainsi la distribution de l'humidité à travers le matelas. Toutefois par ce moyen on ne réduit pas le volume du matelas.

Afin de limiter l'épaisseur nécessaire à celui-ci pour remplir sa fonction, on a proposé de lier les fibres cellulosiques entre elles au moyen de fibres thermofusibles que l'on incorpore dans la masse au moment de la constitution du matelas et que l'on fait fondre de façon à faire adhérer les fibres cellulosiques entre elles par ce liant.

L'invention se propose de réaliser un matelas assurant à la fois une bonne distribution des liquides, une surface de captation la plus large possible, au moins dans la zone de tronc avant susceptible de recueillir les liquides, tout en étant de réalisation très économique.

Conformément à l'invention un change complet à usage unique du type comprenant un matelas absorbant inséré entre une feuille imperméable et une feuille perméable aux liquides, le change présentant dans le sens longitudinal une zone d'entrejambe prolongée de part et d'autre par une zone de tronc avant et une zone de tronc arrière, ledit matelas étant constitué d'un matériau absorbant composé essentiellement de fibres hydrophiles liées entre elles de manière à former une masse cohérente couvrant une partie étroite correspondant à la zone d'entrejambe s'élargissant vers les zones de tronc avant et arrière ledit change étant caractérisé en ce que le matelas est composé, au moins dans l'une des zones de tronc, d'au moins trois branches disposées longitudinalement dans le prolongement de ladite partie étroite en s'écartant les unes des autres de manière à ménager des cavités entre elles et les deux feuilles interne et externe.

De préférence le matelas comportera plus de trois branches s'étendant depuis la zone centrale d'entrejambe. Leur nombre dépendra de l'application et de la taille du change. Le nombre sera de préférence impair afin que l'une des branches soit disposée dans l'axe longitudinal du change. De plus, cette branche pourra être plus large que les autres. On fera en sorte que l'espace laissé entre deux branches adjacentes ne soit pas trop important, et que la largeur des branches ne soit pas trop réduite compte tenu de la solidité du matelas.

Afin d'améliorer la capacité d'absorption, il est possible d'incorporer dans la masse des particules de matières connues sous le nom de superabsorbants. Il s'agit de composés insolubles dans l'eau mais qui gonflent en présence de liquide qu'ils peuvent absorber en quantité correspondant à plusieurs dizaines de fois leur poids à sec. Leur intérêt évident dans la présente application a conduit les fabricants d'articles d'hygiène à usage unique à les mélanger aux fibres cellulosiques. Ils présentent cependant l'inconvénient de former barrage après un premier gonflement, si bien que le liquide glisse sur la masse déjà humectée en surface sans pouvoir pénétrer dans les couches plus profondes du matelas. En fractionnant le matelas comme cela est proposé par l'invention, on augmente la surface effective en contact immédiat avec le liquide. Celui-ci est absorbé à travers la surface en contact avec la peau de l'utilisateur mais aussi à travers les portions de surface disposées transversalement. On augmente ainsi la vitesse d'absorption ainsi que l'on améliore la capacité d'absorption du matelas après une première humectation.

La disposition du matelas en branches ou ramifications longitudinales étalées en éventail assure également une surface de captation des liquides maximale pour une quantité de matière utilisée minimale et permet de minimiser les coûts de production. En effet, pour un change de l'art antérieur on part d'un matelas de fibres de largeur égale à celle des zones de tronc que l'on découpe latéralement pour former la zone d'entrejambe. Pour réaliser le change conforme à l'invention on part au contraire d'un matelas de largeur égale à celle de la zone d'entrejambe dont on dispose les ramifications en éventail pour former les zones de tronc. Ainsi, non seulement on ne produit pas de chutes mais encore on utilise moins de matériau pour couvrir la même surface.

Par ailleurs, les branches constituent autant de barrages pour les écoulements ou infiltrations dirigés dans le sens transversal par rapport au change, limitant de la sorte les risques de fuites par les côtés. En effet, le problème rencontré avec les matelas de l'art antérieur ne vient pas tant de la capacité d'absorption totale du matelas que l'absorption des liquides avant qu'ils n'aient migré vers les bords du change et souillé les vêtements de l'utilisateur. Par cette disposition, on freine les migrations transversales et on favorise les migrations longitudinales dans le sens des cavités qui forment des drains vers la zone centrale d'entrejambe du matelas.

Enfin, par la réduction du volume de la masse absorbante dans la zone de tronc avant, par rapport aux réalisations connues, le change gagne en souplesse. Le confort de l'utilisateur s'en trouve amélioré et son port devient plus discret sous les vêtements.

D'autres caractéristiques et avantages apparaîtront à la lecture de la description qui suit, d'un mode de réalisation non limitatif de l'invention où:

La figure 1 représente un change complet à jeter après usage.
La figure 2 est une vue en coupe de la vue 1 selon A.A.

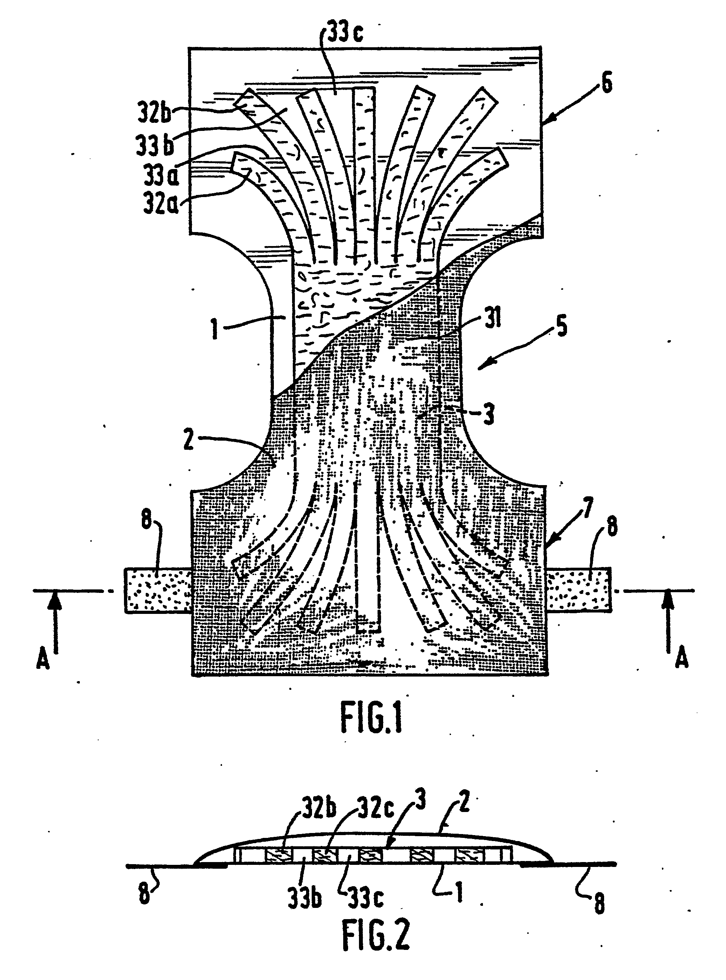

Sur la figure 1 est représenté un change complet à jeter après usage selon l'invention, vu du côté de la face interne en non-tissé, dont on a représenté une partie en arraché afin de montrer le matelas absorbant intérieur.

Le change comporte donc une feuille support 1 en matériau résistant, souple, imperméable aux liquides et constituant la face externe du change pendant le port de ce dernier. Un matériau généralement utilisé est le polyéthylène mais d'autres matériaux plastiques conviendraient également.

Une feuille 2 en matériau perméable aux fluides est disposée sur la feuille support, cette feuille constitue la face interne du change et vient au contact de la peau de l'utilisateur ; elle est réalisée en un matériau non irritant pouvant être aisément traversé par les liquides corporels, de préférence il sera hydrophobe afin de conserver à la feuille un état de surface sec. Il peut s'agir d'un textile nontissé réalisé à partir de fibres obtenues par étirage de matériaux thermoplastiques, par exemple le nontissé vendu sous la marque HOLNEST par la demanderesse. D'autres matériaux présentant les mêmes propriétés sont connus de l'homme de métier. Les deux feuilles sont soudées, ou solidarisées par collage, entre elles au moins bord à bord de manière à former une enveloppe fermée. A l'intérieur de cette enveloppe est enfermé le matelas absorbant 3 de l'invention, destiné à recueillir et contenir les liquides corporels ayant traversé la feuille 2.

De façon connue le matelas est constitué de fibres hydrophiles, notamment de fibres obtenues par défibrage à sec de feuilles de pâte à papier. Pour les besoins de l'invention le matelas a été rendu cohérent, par exemple par mélange, pendant la formation de la nappe, de fibres thermofusibles en quantité suffisante pour permettre, après leur fusion et leur refroidissement, aux fibres cellulosiques d'adhérer les unes aux autres sans perdre de leur capacité d'absorption. Un exemple de réalisation d'une telle structure est décrit dans le brevet FR 2256279.

A l'intérieur du matelas, des particules de superabsorbant ont été également mélangées aux fibres. Les produits les plus connus à cet usage sont les alginates, les carboxyméthyl celluloses réticulés, les amidons greffés, les dérivés de synthèse du type acrylamide ou du type acrylate.

Le change présente une zone centrale d'entrejambe étroite 5, une zone de tronc avant 6 et une zone de tronc arrière 7. Sur la feuille support de cette dernière sont fixés des moyens d'attache 8 constitués de façon connue par des pattes en papier, ou autre matériau, adhésivées sur une face.

Le matelas 3 disposé sur la feuille support, comporte une zone centrale 31, étroite, allant en s'élargissant vers les zones de tronc avant et arrière. Conformément à l'invention, la partie du matelas relative à la zone de tronc avant et éventuellement la zone de tronc arrière est conformée en branches ou ramifications s'écartant les unes des autres 32a, 32b, 32c etc... et ménageant entre elles et les feuilles de couvertures 1 et 2, des cavités 33a, 33b, etc... Ces cavités ont une forme effilée vers la zone centrale. Le nombre de branches 32 à prévoir, est au moins égal à trois, mais il dépendra essentiellement de l'application et de la taille du change. Il pourra être choisi entre 3 et 19, de préférence 3 et 11, s'il est impair, l'une des branches sera disposée dans l'axe longitudinal du change. Elle pourra être alors plus large que les autres.

Un moyen simple de réaliser un matelas présentant la caractéristique selon l'invention, consiste à préparer un matelas absorbant en forme d'une bande de largeur égale à celle de la zone d'entrejambe 31, dans la pratique entre 8 et 25 cm, et de longueur correspondant à la longueur prévue pour le matelas, 70 cm par exemple. Le matelas étant cohérent, il est possible de pratiquer dans le sens longitudinal des entailles de longueur déterminée à partie d'une extrémité du ruban, ou des deux extrémités si on désire réaliser l'invention pour les deux zones de tronc ; on obtient de la sorte des branches 32 rattachées à une partie non découpée 31. La longueur des branches correspond sensiblement à celle des zones de tronc. Leur largeur dépendra de leur nombre, par example, pour un matelas large de 15 cm on peut découper 5 branches de 3 cm chacune, ou 10 branches de 1,5 cm.

Le matelas ainsi préparé, est déposé sur une feuille support. Les lamelles sont étalées en éventail de manière à couvrir la surface désirée. Pour reprendre l'exemple précédent, on forme un éventail dont la plus grande largeur est inférieure à 50 cm, de préférence 40 cm. L'espacement 33 peut être le même entre toutes les ramifications, il peut être non uniforme, par exemple il est possible de resserrer les ramifications centrales. Le rapport de la plus grande largeur à la plus petite largeur de l'éventail ainsi réalisé sera toutefois inférieur à cinq, de préférence compris entre 2 et 4. Après sa mise en place, le matelas est maintenu sur la feuille au moyen d'un adhésif ou par soudage. La fabrication du change ne présente pour le reste aucune particularité.

En usage, les liquides traversent la feuille perméable 2, sont en partie absorbés directement par les branches de la zone de tronc avant en y pénétrant par la face disposée du côté de la peau de l'utilisateur et les faces latérales. Les cavités 33 assurent un écoulement rapide le long de ces branches mettant les liquides en contact avec le matelas sur une grande surface pour une absorption rapide et étendue, éventuellement les liquides parviennent jusqu'à la zone centrale pour leur absorption totale.

Grâce à la disposition longitudinale, chacune des branches forme barrage à une migration des liquides dans le sens transversal, la cavité correspondante les ramenant toujours dans le sens longitudinal.

L'invention n'est pas limitée au mode de réalisation décrit, elle englobe notamment tous les équivalents à la portée de l'homme de métier.

## Revendications

1. Change complet à usage unique du type comprenant un matelas absorbant inséré entre une feuille (1) imperméable aux liquides et une feuille perméable (2), le change présentant dans le sens longitudinal une zone d'entrejambe (5) prolongée de part et d'autre par une zone de tronc avant (6) et une zone de tronc arrière (7), ledit matelas (3) étant constitué d'un matériau absorbant composé essentiellement de fibres hydrophiles liées entre elles de manière à former une structure cohérente couvrant une partie étroite (31) correspondant à la zone d'entrejambe (5), et s'élargissant vers les zones de tronc avant et arrière, caractérisé en ce que le matelas est composé, au moins dans l'une des zones de tronc, d'au moins trois branches (32) disposées longitudinalement dans le prolongement de la partie étroite (31) en s'écartant les unes des autres de manière à ménager des cavités entre elles et les feuilles (1) et (2).

2. Change selon la revendication 1, caractérisé en ce que le matelas (3) est composé dans la zone de tronc de moins de dix neuf branches (32).

3. Change selon l'une des revendications précédentes, caractérisé en ce que l'une des branches (32) est disposée dans l'axe longitudinal du matelas, et sa largeur est supérieure à celle des branches adjacentes.

4. Change selon l'une des revendications précédentes, caractérisé en ce que la largeur totale des branches (32) est au plus égale à celle de la partie étroite (31) du matelas.

5. Change selon l'une des revendications précédentes, caractérisé en ce que des particules de produit superabsorbant sont incorporées dans la masse absorbante.

6. Procédé pour réaliser un change selon l'une des revendications précédentes, caractérisé en ce qu'il comprend les étapes suivantes :
- préparation d'un matelas absorbant de forme sensiblement rectangulaire
- formation dans le sens longitudinal du matelas, d'au moins deux entailles de longueur déterminée à partir d'au moins une des extrémités longitudinales
- écartement des branches ainsi réalisées, les unes des autres en un éventail dont le rapport entre sa plus grande largeur et sa plus petite largeur est compris entre 1 et 5. Cette partie en éventail devant constituer l'une des zones de tronc dudit change

7. Procédé selon la revendication 6, caractérisé en ce que ledit rapport est compris entre 2 et 4.

8. Procédé selon l'une des revendications 6 et 7, caractérisé en ce que la largeur du matelas rectangulaire est comprise entre 8 et 25 cm.

9. Procédé selon l'une des revendications 6 à 8, caractérisé en ce que la largeur de l'éventail réalisé est inférieure à 50 cm.

## Claims

1. Disposable all-in-one nappy of the type comprising an absorbent pad inserted between a sheet (1) which is impermeable to liquids and a sheet (2) which is permeable to liquids, the nappy having in the longitudinal direction a crutch region (5) extended on either side by a front trunk region (6) and a rear trunk region (7), the pad (3) being formed of an absorbent material composed essentially of hydrophilic fibres joined together so as to form a coherent mass covering a narrow portion (31) corresponding to the crutch region (5), which widens towards the front and rear trunk regions, characterised in that the pad is composed, at least in one of the trunk regions, of at least three branches (32) arranged longitudinally in the extension of said narrow portion (31) and spreading out from one another so as to form cavities between them and sheets (1) and (2).

2. Nappy according to claim 1, characterised in that the pad (3) is composed in the trunk region of at least nine branches (32).

3. Nappy according to one of the preceding claims, characterised in that one of the branches (32) lies in the longitudinal axis of the pad, and its width is larger than that of the adjacent branches.

4. Nappy according to one of the preceding claims, characterised in that the total width of the branches (32) is at the most equal to that of the narrow part (31) of the pad.

5. Nappy according to one of the preceding claims, characterised in that particles of a superabsorbent substance are incorporated in the absorbent mass.

6. A process for manufacturing a nappy according to one of the preceding claims, characterised in that it comprises the following stages:
- preparation of an absorbent pad of substantially rectangular shape
- formation in the longitudinal direction of the pad of at least two slots of specified length, starting from at least one of the longitudinal ends
- spacing of the branches thus formed so as to form a fan, whose ratio between its largest and smallest width is between 1 and 5. This fan-shaped portion must form one of the trunk regions of the nappy.

7. Process according to claim 6, characterised in that the ratio is between 2 and 4.

8. Process according to one of claims 6 and 7, characterised in that the width of the rectangular pad is between 8 and 25 cm.

9. Process according to one of claims 6 to 8, characterised in that the width of the fan is less than 50 cm.


**Patentansprüche**

1. Wegwerfwindel für den Einmalgebrauch mit einem absorbierenden Kissen, das zwischen einer Folie (1), die flüssigkeitsundurchlässig ist, und einer durchlässigen Folie (2) angeordnet ist, wobei die Windel in Längsrichtung gesehen einen Zwischenbeinbereich (5) aufweist, der zu beiden Enden durch einen vorderen Rumpfbereich (6) und einen hinteren Rumpfbereich (7) verlängert wird, und das Kissen (3) aus einem im wesentlichen aus hydrophilen Fasern bestehendem Material aufgebaut ist, die untereinander derart verbunden sind, daß sie eine zusammenhängende Struktur bilden, die einen schmalen Bereich (31) abdeckt, der dem Zwischenbeinbereich (5) entspricht und die sich zu dem vorderen und dem hinteren Rumpfbereich verbreitert, dadurch gekennzeichnet, daß das Kissen in mindestens einer der Rumpfzonen aus mindestens drei Ästen (32) aufgebaut ist, die in Längsrichtung in der Verlängerung des schmalen Bereiches (31) ausgebildet sind und sich einer von dem anderen entfernen derart, daß sie zwischen sich und den Folien (1) und (2) Aushöhlungen bilden.

2. Windel nach Anspruch 1, dadurch gekennzeichnet, daß das Kissen (3) im Rumpfbereich aus mindestens 19 Ästen (32) aufgebaut ist.

3. Windel nach einem der vorhergehenden Ansprüche, dadurch gekennzeichnet, daß einer der Äste (32) in der Längsachse des Kissens ausgerichtet ist und daß die Breite dieses Astes größer als die der benachbarten Äste ist.

4. Windel nach einem der vorhergehenden Ansprüche, dadurch gekennzeichnet, daß die Gesamtbreite der Äste (32) höchstens gleich der Breite des schmalen Kissenbereiches (31) ist.

5. Windel nach einem der vorhergehenden Ansprüche, dadurch gekennzeichnet, daß die Partikel des Produktes mit superabsorbierenden Eigenschaften in die absorbierende Masse eingearbeitet sind.

6. Verfahren zur Herstellung einer Windel nach einem der vorhergehenden Ansprüche, gekennzeichnet durch folgende Schritte:
- Herstellung eines im wesentlichen rechteckigen absorbierenden Kissens
- Ausbildung in Längsrichtung des Kissens von mindestens zwei Einschnitten mit vorherbestimmter Länge, die von mindestens einem der Längsenden ausgehen
- Auseinanderspreizen der derart hergestellten Äste voneinander in Form eines Fächers, bei dem das Verhältnis zwischen seiner größten Breite und seiner schmalsten Breite zwischen 1 und 5 liegt, wobei dieser fächerförmige Teil einen der Rumpfbereiche der Windel bilden muß.

7. Verfahren nach Anspruch 6, dadurch gekennzeichnet, daß das Verhältnis zwischen 2 und 4 liegt.

8. Verfahren nach einem der Ansprüche 6 und 7, dadurch gekennzeichnet, daß die Breite des rechteckigen Kissens zwischen 8 und 25 cm beträgt.

9. Verfahren nach einem der Ansprüche 6 bis 8, dadurch gekennzeichnet, daß die Breite des hergestellten Fächers geringer als 50 cm ist.

FIG.1

FIG.2